# EUROPEAN PATENT APPLICATION

(11) **EP 4 566 607 A1**
(43) Date of publication of application: **11.06.2025**
(21) Application number: 23849348.0
(22) Date of filing: 31.07.2023
(51) Int. Cl.: A61K 31/4439, A61K 9/20, C07D 413/12, A61P 19/06, A61P 35/00, A61P 37/02, A61P 3/00, A61P 9/10, A61P 43/00, A61P 1/00, A61P 19/02, A61P 11/06, A61P 37/08

(54) **IRAK4 INHIBITOR COMPOSITION, PREPARATION METHOD THEREFOR AND USE THEREOF**

(30) Priority: 01.08.2022 CN 202210917341
(71) Applicant: Wuhan Createrna Science and Technology Co.,Ltd., Wuhan, Hubei 430075 (CN)
(72) Inventor: LUAN, Linbo, Shanghai 200120 (CN); TIAN, Yong, Shanghai 200120 (CN)
(74) Representative: Jones Day
(86) International application number: PCT/CN2023/110206
(87) International publication number: WO 2024/027641

(57) **Abstract**

An IRAK4 inhibitor composition, a preparation method and use thereof. The composition comprises: (i) a compound of formula I, a stereoisomer, a solvate or a pharmaceutically acceptable salt thereof; (ii) a diluent; (iii) a disintegrant; and (iv) a lubricant. The structure of formula I is shown as follows:

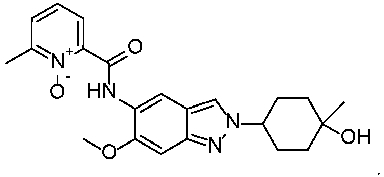

## Description

The present application claims priority to the prior application filed with the China National Intellectual Property Administration on August 1, 2022 with the patent application number 202210917341.X and entitled "IRAK4 inhibitor Composition and Preparation Method and Use Thereof." The entirety of that application is incorporated herein by reference.

### TECHNICAL FIELD

The present invention belongs to the field of pharmaceutical preparations, and in particular relates to an IRAK4 inhibitor composition and a preparation method and use thereof.

### BACKGROUND

Interleukin-1 receptor-associated kinases (IRAK) are a family of serine/threonine protein kinases present in cells. There are four members: IRAK1, IRAK2, IRAK-M, and IRAK4 with the common feature being that they have a typical N-terminal death domain that mediates the interaction with the MyD88-family adaptor protein and the kinase domain located in the center, wherein IRAK1 and IRAK4 have kinase activities. IRAK4 is a key factor downstream of the inflammatory signal transduction pathway mediated by Toll-like receptor (TLR)/interleukin-1 receptor (IL-1R). The extracellular part of TLR recognizes pathogen-specific molecules (such as lipopolysaccharide, polypeptides, viral DNA, etc.). After binding to the ligand, the intracellular part recruits MyD88 and others to form a complex, activating IRAK1 autophosphorylation, and then activating the downstream serine/threonine kinase TAK1, activating NF-κB and MAPK signaling pathways, subsequently producing proinflammatory cytokines, chemokines, and destructive enzymes, and ultimately leading to an inflammatory response and mediating innate immunity. IL-1R is involved in host defense and hematopoiesis, and serves as a bridge between innate and adaptive immunity.

WO2021057785A1 discloses an IRAK inhibitor compound, including a compound shown in Formula I. At present, no document has disclosed a stable pharmaceutical formulation of the compound of formula I.

### SUMMARY

In order to solve the problems in the prior art, the object of the present invention is to provide a pharmaceutical composition with good dissolution, low impurity content, and good stability.

Therefore, the present invention relates in one aspect to a pharmaceutical composition, comprising:
(i) a compound of formula I, a stereoisomer, a solvate, or a pharmaceutically acceptable salt thereof, wherein the structure of formula I is as follows: 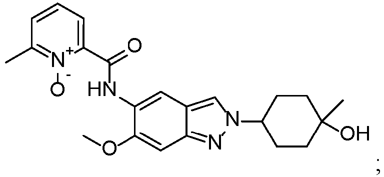
(ii) a diluent;
(iii) a disintegrant; and
(iv) a lubricant.

According to an embodiment of the present invention, the composition may further optionally comprise at least one of (v) a glidant and (vi) a surfactant.

According to an embodiment of the present invention, the diluent is selected from at least one of microcrystalline cellulose, anhydrous calcium hydrogen phosphate, and lactose. Preferably, the diluent is selected from at least one of anhydrous calcium hydrogen phosphate and microcrystalline cellulose. In some implementations, the diluent is a combination of anhydrous calcium hydrogen phosphate and microcrystalline cellulose. Preferably, the mass ratio of anhydrous calcium hydrogen phosphate to microcrystalline cellulose is 1:10 to 10:1, for example, 1:1, 1:2, 1:3, 1:4, 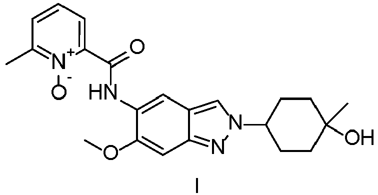 1:5, 1:6, 1:7, 1:8, 1:9, 1:10, 2:1, 3:1, 4:1, 5:1, 6:1, 7:1, 8:1, 9:1, or 10:1; preferably, 2:1 to 6:1, and more preferably, 3:1.

According to an embodiment of the present invention, based on the total weight of the composition, the content of the diluent may be any value in a range from 18% to 90% or a range formed by a combination of any values within the range, for example, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46% , 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, etc.

According to an embodiment of the present invention, the glidant is selected from colloidal silicon dioxide. Based on the total weight of the composition, the content of the glidant may range from 0.1% to 5%. Based on the total weight of the composition, the content of the glidant may be any value in a range from 0.1% to 5% or a range formed by a combination of any values within the range, for example, 0.1%, 0.2%, 0.3%, 0.4%, 0.5%, 0.6%, 0.7%, 0.8%, 0.9%, 1.0%, 1.2%, 1.4%, 1.6%, 1.8%, 2.0%, 2.2%, 2.4%, 2.6%, 2.8%, 3.0%, 3.2%, 3.4%, 3.6%, 3.8%, 4.0%, 4.2%, 4.4%, 4.6%, 4.8%, 5.0%, etc.

According to an embodiment of the present invention, the disintegrant is selected from at least one of cross-linked sodium carboxymethyl cellulose, sodium carboxymethyl starch, hydroxypropyl cellulose, cross-linked polyvinyl pyrrolidone and cross-linked povidone, preferably at least one of cross-linked sodium carboxymethyl cellulose and sodium carboxymethyl starch, and more preferably sodium carboxymethyl starch. Based on the total weight of the composition, the content of the disintegrant may be any value in a range from 1% to 10% or a range formed by a combination of any values within the range, for example, 1%, 2%, 3%, 4%, 5%, 6%, 7%, 8%, 9%, 10%, etc.

According to an embodiment of the present invention, the surfactant is selected from at least one of sodium dodecyl sulfate, Tween 80, poloxamer, and potassium oleate, preferably sodium dodecyl sulfate. Based on the total weight of the composition, the content of the surfactant may be any value in a range from 1% to 5% or a range formed by a combination of any values within the range, for example,1%, 2%, 3%, 4%, 5%, etc.

According to an embodiment of the present invention, the lubricant is selected from at least one of magnesium stearate, talc, sodium stearyl fumarate, zinc stearate, sodium lauryl sulfate, and hydrogenated vegetable oil, preferably magnesium stearate. Based on the total weight of the composition, the content of the lubricant may be any value in a range from 0.1% to 5% or a range formed by a combination of any values within the range, for example, 0.1%, 0.2%, 0.3%, 0.4%, 0.5%, 0.6%, 0.7%, 0.8%, 0.9%, 1.0%, 1.2%, 1.4%, 1.6%, 1.8%, 2.0%, 2.2%, 2.4%, 2.6%, 2.8%, 3.0%, 3.2%, 3.4%, 3.6%, 3.8%, 4.0%, 4.2%, 4.4%, 4.6%, 4.8%, 5.0%, etc.

According to an embodiment of the present invention, the compound of formula I is selected from the following structure:

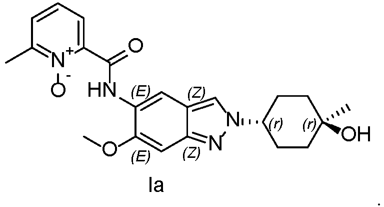

According to an embodiment of the present invention, the pharmaceutically acceptable salt may be, for example, an acid addition salt of a compound of the present invention having a nitrogen atom in a chain or ring and having sufficient alkalinity; an acid addition salt that can be formed with the following inorganic acids: for example, hydrochloric acid, hydrofluoric acid, hydrobromic acid, hydroiodic acid, sulfuric acid, pyrosulfuric acid, phosphoric acid or nitric acid, or disulfate; or an acid addition salt formed with the following organic acids: for example, formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoric acid, cinnamic acid, cyclopentylpropionic acid, digluconic acid, 3-hydroxy-2-naphthoic acid, nicotinic acid, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptanoic acid, glycerophosphoric acid, aspartic acid, sulfosalicylic acid, hemisulfuric acid, or thiocyanic acid.

According to an embodiment of the present invention, the "solvate" refers to an associated complex formed by one or more solvent molecules and the compound of the present invention. Solvents that form solvates include, but are not limited to, water, isopropanol, ethanol, methanol, dimethyl sulfoxide, ethyl acetate, acetic acid, and aminoethanol.

According to an embodiment of the present invention, the compound of formula I may be in different crystalline forms, for example, any crystalline form disclosed in CN202210267507.8 may be adopted.

In some implementations, the compound of formula I is in the form of crystalline form III, and the crystalline form III has the X-ray powder diffraction expressed as 2θ angles comprising characteristic peaks at 12.15±0.20°, 15.98±0.20°, 16.62±0.20°, 17.14±0.20°, 24.32±0.20°, and 26.08±0.20° using Cu-Kα radiation.

According to an embodiment of the present invention, the crystalline form III is an anhydrate of compound A.

Preferably, the crystalline form III has the X-ray powder diffraction expressed as 2θ angles comprising characteristic peaks at 12.15±0.20°, 15.04±0.20°, 15.98±0.20°, 16.62±0.20°, 17.14±0.20°, 21.09±0.20°, 24.32±0.20°, and 26.08±0.20° using Cu-Kα radiation.

Preferably, the crystalline form III has the X-ray powder diffraction expressed as 2θ angles comprising characteristic peaks at 12.15±0.20°, 15.04±0.20°, 15.98±0.20°, 16.62±0.20°, 17.14±0.20°, 18.74±0.20°, 21.09±0.20°, 23.51±0.20°, 24.32±0.20°, and 26.08±0.20° using Cu-Kα radiation.

Preferably, the crystalline form III has the X-ray powder diffraction expressed as 2θ angles and shown in Table 1 using Cu-Kα radiation, with an error range of ±0.20°:

**Table 1 XRPD analysis data of crystalline form III**

| Peak No. | 2θ [ °] | Relative Intensity % | Peak No. | 2θ [ °] | Relative Intensity % |
|---|---|---|---|---|---|
| 1 | 6.021 | 5.2 | 22 | 26.083 | 37.3 |
| 2 | 10.879 | 8.2 | 23 | 26.688 | 1.3 |
| 3 | 12.152 | 77.4 | 24 | 27.106 | 1 |
| 4 | 12.978 | 3.6 | 25 | 27.83 | 5.8 |
| 5 | 15.04 | 20.5 | 26 | 28.262 | 0.2 |
| 6 | 15.986 | 47.8 | 27 | 29.314 | 9.1 |
| 7 | 16.617 | 34.2 | 28 | 29.824 | 3.2 |
| 8 | 17.141 | 27 | 29 | 30.429 | 3.6 |
| 9 | 18.323 | 1.5 | 30 | 30.818 | 1.5 |
| 10 | 18.742 | 13.7 | 31 | 31.926 | 4.9 |
| 11 | 20.068 | 8.7 | 32 | 32.373 | 3.1 |
| 12 | 20.449 | 3.5 | 33 | 32.583 | 2.3 |
| 13 | 20.765 | 3.2 | 34 | 33.186 | 1.4 |
| 14 | 21.092 | 21.8 | 35 | 33.777 | 2.5 |
| 15 | 21.645 | 2.2 | 36 | 34.392 | 0.6 |
| 16 | 22.262 | 5.1 | 37 | 35.418 | 2.4 |
| 17 | 22.97 | 0.4 | 38 | 36.023 | 1.7 |
| 18 | 23.51 | 19.6 | 39 | 36.6 | 2 |
| 19 | 24.048 | 100 | 40 | 38.138 | 4 |
| 20 | 24.323 | 50.8 | 41 | 38.806 | 4.3 |
| 21 | 24.598 | 8.3 | 42 | 39.306 | 2 |

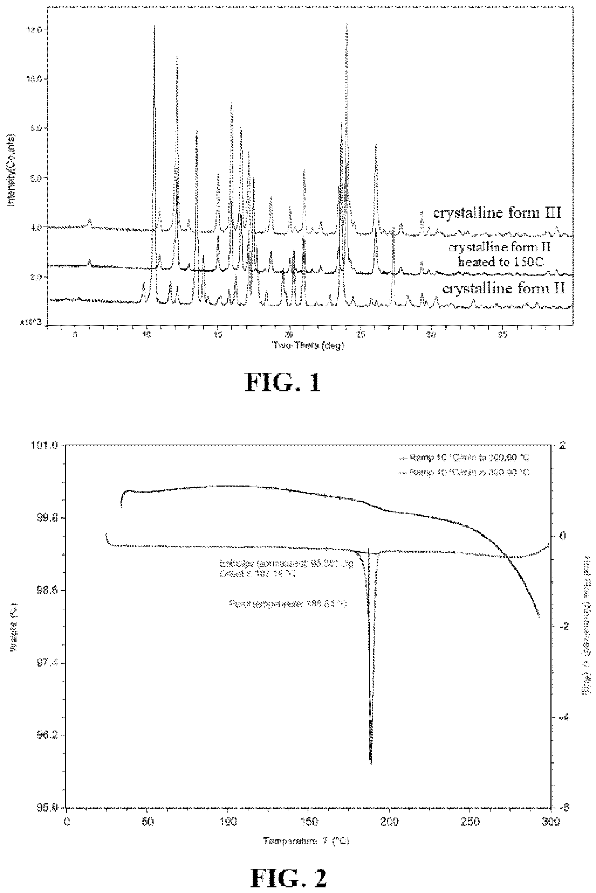

Preferably, the crystalline form III has a powder X-ray diffraction pattern substantially as shown in FIG. 1.

According to an embodiment of the present invention, differential scanning calorimetry (DSC) analysis of the crystalline form III shows that a first endothermic peak appears when being heated near the peak temperature of 188.81°C.

According to an embodiment of the present invention, thermogravimetric analysis (TGA) of the crystalline form III shows almost no weight loss before 180°C.

Preferably, the crystalline form III has a DSC-TGA pattern substantially as shown in FIG. 2.

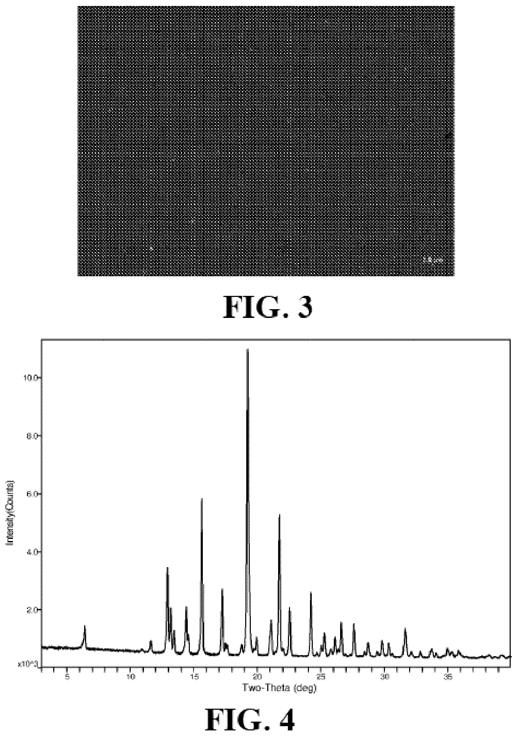

According to an embodiment of the present invention, the crystal form III is a crystal with irregular morphology. Preferably, the particle size of the crystalline form III is less than 5 µm. Preferably, the crystalline form III has a PLM pattern substantially as shown in FIG. 3.

In some implementations, the compound A is in the form of crystalline form VII, and the crystalline form VII has the X-ray powder diffraction expressed as 2θ angles comprising characteristic peaks at 12.94±0.20°, 14.41±0.20°, 15.64±0.20°, 17.25±0.20°, 21.75±0.20°, and 24.23±0.20° using Cu-Kα radiation.

According to an embodiment of the present invention, the crystalline form VII is an anhydrate of the compound A.

Preferably, the crystalline form VII has the X-ray powder diffraction expressed as 2θ angles comprising characteristic peaks at 12.94±0.20°, 13.18±0.20°, 14.41±0.20°, 15.64±0.20°, 17.25±0.20°, 21.75±0.20°, 22.54±0.20°, and 24.23±0.20° using Cu-Kα radiation.

Preferably, the crystalline form VII has the X-ray powder diffraction expressed as 2θ angles comprising characteristic peaks at 12.94±0.20°, 13.18±0.20°, 14.41±0.20°, 15.64±0.20°, 17.25±0.20°, 21.11±0.20°, 21.75±0.20°, 22.54±0.20°, 24.23±0.20°, 26.62±0.20°, and 31.64±0.20° using Cu-Kα radiation.

Preferably, the crystalline form VII has the X-ray powder diffraction expressed as 2θ angles and shown in Table 2 using Cu-Kα radiation, with an error range of ±0.20°:

**Table 2 XRPD analysis data of crystalline form VII**

| Peak No. | 2θ[ ° ] | Relative Intensity % | Peak No. | 2θ [ ° ] | Relative Intensity % |
|---|---|---|---|---|---|
| 1 | 6.413 | 8 | 25 | 25.82 | 1.7 |
| 2 | 10.957 | 1.1 | 26 | 26.135 | 6.1 |
| 3 | 11.625 | 3.1 | 27 | 26.385 | 3 |
| 4 | 12.939 | 25.9 | 28 | 26.621 | 10.5 |
| 5 | 13.176 | 12.7 | 29 | 27.328 | 0.2 |
| 6 | 13.451 | 5.9 | 30 | 27.618 | 9.2 |
| 7 | 14.409 | 16.3 | 31 | 28.436 | 2 |
| 8 | 14.567 | 9.9 | 32 | 28.724 | 4.6 |
| 9 | 15.644 | 41.4 | 33 | 29.431 | 1.4 |
| 10 | 17.246 | 18.2 | 34 | 29.825 | 5.9 |
| 11 | 17.522 | 6.2 | 35 | 30.35 | 4.9 |
| 12 | 17.655 | 4.4 | 36 | 30.624 | 0.8 |
| 13 | 18.783 | 1.4 | 37 | 31.637 | 11.1 |
| 14 | 19.255 | 100 | 38 | 32.123 | 2.6 |
| 15 | 19.732 | 3.4 | 39 | 32.856 | 1.5 |
| 16 | 19.94 | 5.1 | 40 | 33.763 | 3.9 |
| 17 | 21.106 | 10.3 | 41 | 34.092 | 1.4 |
| 18 | 21.75 | 41 | 42 | 34.973 | 4.4 |
| 19 | 22.001 | 3.4 | 43 | 35.367 | 2.8 |
| 20 | 22.539 | 12.6 | 44 | 35.841 | 2.9 |
| 21 | 24.231 | 18 | 45 | 37.7 | 1.2 |
| 22 | 24.704 | 0.7 | 46 | 38.242 | 1.6 |
| 23 | 25.058 | 3.6 | 47 | 39.185 | 2.1 |
| 24 | 25.308 | 7 | 48 | 39.307 | 2.1 |

Preferably, the crystalline form VII has a powder X-ray diffraction pattern substantially as shown in FIG. 4.

According to an embodiment of the present invention, differential scanning calorimetry (DSC) analysis of the crystalline form VII shows that a first endothermic peak appears when being heated near the peak temperature of 201.07°C.

According to an embodiment of the present invention, thermogravimetric analysis (TGA) of the crystalline form VII shows almost no weight loss before 200°C, for example, almost no weight loss before 180°C.

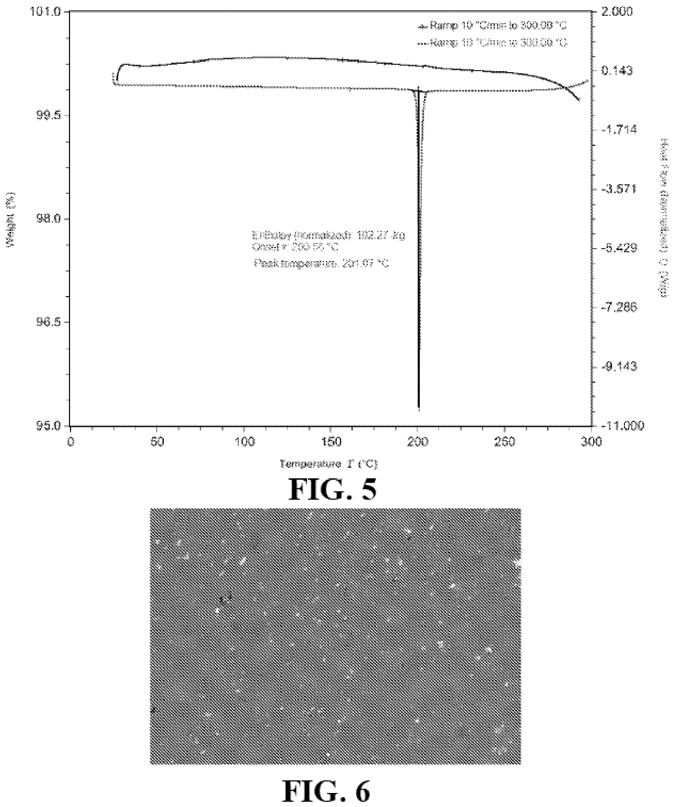

Preferably, the crystalline form VII has a DSC-TGA pattern substantially as shown in FIG. 5.

According to an embodiment of the present invention, the crystal form VII is a crystal with irregular morphology. Preferably, the particle size of the crystalline form VII is less than 5 µm. Preferably, the crystalline form VII has a PLM pattern substantially as shown in FIG. 6.

In some implementations, the pharmaceutical composition comprises the following components by weight percentage:
a compound of formula I 5-15%
anhydrous calcium hydrogen phosphate 55-65%
microcrystalline cellulose 15-25%
sodium dodecyl sulfate 0.5-5%
colloidal silicon dioxide 0.5-2%
cross-linked sodium carboxymethyl cellulose or sodium carboxymethyl starch 1-5%
magnesium stearate 0.5-5%.

In some implementations, the pharmaceutical composition comprises the following components by weight percentage:
the compound of formula I 8-12%
anhydrous calcium hydrogen phosphate 60-65%
microcrystalline cellulose 18-25%
sodium dodecyl sulfate 0.5-3%
colloidal silicon dioxide 0.5-1.5%
cross-linked sodium carboxymethyl cellulose 2.5-3.5%
magnesium stearate 0.5-1.5%.

In some implementations, the pharmaceutical composition comprises the following components by weight percentage:
the compound of formula I 10%
anhydrous calcium hydrogen phosphate 63%
microcrystalline cellulose 21%
sodium dodecyl sulfate 1%
colloidal silicon dioxide 1%
cross-linked sodium carboxymethyl cellulose 3%
magnesium stearate 1%.

In some implementations, the pharmaceutical composition comprises the following components by weight percentage:
the compound of formula I 8-12%
anhydrous calcium hydrogen phosphate 60-63%
microcrystalline cellulose 18-25%
sodium dodecyl sulfate 2.5-3.5%
colloidal silicon dioxide 0.5-1.5%
sodium carboxymethyl starch 2.5-3.5%
magnesium stearate 0.5-1.5%.

In some implementations, the pharmaceutical composition comprises the following components by weight percentage:
the compound of formula I 10%
anhydrous calcium hydrogen phosphate 61.5%
microcrystalline cellulose 20.5%
sodium dodecyl sulfate 3%
colloidal silicon dioxide 1%
sodium carboxymethyl starch 3%
magnesium stearate 1%.

According to an embodiment of the present invention, the pharmaceutical composition may be an oral preparation selected from tablets, capsules, mini-tablets, or granules, preferably tablets.

In some implementations, the pharmaceutical composition of the present invention is a tablet, which includes a core and a coating, and the coating material is selected from at least one of hydroxypropyl methylcellulose, polyvinyl alcohol, hydroxypropyl cellulose, polyacrylic acid resin, and Opadry, preferably Opadry (for example, Opadry I and Opadry II). The coating mass accounts for 1-5% of the total mass of the tablet, preferably 1-4%, and more preferably 2%.

In another aspect, the present invention relates to a preparation method of a pharmaceutical composition, comprising: mixing the compound of formula I with other components and tableting.

According to an embodiment of the present invention, the preparation method further comprises a coating step.

According to an embodiment of the present invention, the preparation method further comprises the following steps:
(1) passing a disintegrant, a lubricant, and a portion of a diluent through a 20-60-mesh sieve;
(2) passing the compound of formula I or optionally the compound together with a glidant and a surfactant through a 40-100-mesh sieve to obtain a premix material 1; then, adding the remaining diluent and the premixed material 1 into a hopper and mixing, and then passing the mixture through a 40-100-mesh sieve to obtain a premixed material 2;
(3) placing, in sequence, the disintegrant, the diluent, and the premixed material 2 that have passed through a 20-60-mesh sieve in a hopper mixer, and mixing;
(4) adding the lubricant that has passed through a 20-60-mesh sieve into the hopper mixer and mixing; and
(5) placing the total mixed powder in a tablet press for tableting.

According to an embodiment of the present invention, the preparation method comprises the following steps: (1) weighing a compound of formula I, sodium dodecyl sulfate, colloidal silicon dioxide, sodium carboxymethyl starch, and magnesium stearate; wherein microcrystalline cellulose is divided into two portions and weighed; anhydrous calcium hydrogen phosphate is divided into two portions and weighed, wherein one portion is weighed at 1/3 of the formulation amount, and the other portion is weighed at 2/3 of the formulation amount;
(2) passing 2/3 of anhydrous calcium hydrogen phosphate, 1/2 of microcrystalline cellulose, sodium carboxymethyl starch, and magnesium stearate through a 40-mesh sieve respectively;
(3) mixing the compound of formula I, colloidal silicon dioxide, and sodium dodecyl sulfate, and then passing the mixture through a 60-mesh sieve to obtain a premix material 1, then, adding 1/2 of the microcrystalline cellulose, 1/3 of anhydrous calcium hydrogen phosphate, and the premixed material 1 into a hopper, mixing, and passing the mixture through a 60-mesh sieve to obtain a premixed material 2;
(4) placing, in sequence, 1/2 of the microcrystalline cellulose, the premixed material 2, sodium carboxymethyl starch, and 2/3 of anhydrous calcium hydrogen phosphate in a hopper mixer, and mixing;
(5) adding magnesium stearate into the hopper mixer and mixing; and
(6) placing the total mixed powder in a tablet press for tableting.

According to an embodiment of the present invention, the preparation method includes adding film coating premix (gastric soluble) Opadry; and preparing an Opadry coating powder suspension with a solid content of 12% for coating.

In another aspect, the present invention relates to use of the pharmaceutical composition in the preparation of a medicament for preventing and/or treating a disease or condition mediated by IRAK.

According to an embodiment of the present invention, the IRAK-mediated disease or condition is selected from diseases such as tumors, gout, systemic lupus erythematosus, multiple sclerosis, metabolic syndrome, atherosclerosis, myocardial infarction, pyaemia, inflammatory bowel disease, rheumatoid arthritis, asthma, and allergies.

In another aspect, the present invention relates to use of the pharmaceutical composition in the preparation of a medicament for preventing and/or treating a disease or condition mediated by interleukin-1 receptor-associated kinases.

According to an embodiment of the present invention, the interleukin-1 receptor-associated kinase mediated disease or condition is selected from diseases such as tumors, gout, systemic lupus erythematosus, multiple sclerosis, metabolic syndrome, atherosclerosis, myocardial infarction, sepsis, inflammatory bowel disease, asthma, rheumatoid arthritis, psoriasis, pyaemia, autoimmune diseases, and allergies.

In another aspect, the present invention relates to a method for preventing and/or treating an IRAK-mediated disease or condition, comprising administering a therapeutically effective amount of the pharmaceutical composition to an individual in need thereof.

In some implementations, the IRAK is selected from IRAK4-related kinases.

In another aspect, the present invention relates to a method for preventing and/or treating an interleukin-1 receptor-associated disease or condition, comprising administering a therapeutically effective amount of the pharmaceutical composition to an individual in need thereof.

Unless otherwise stated, the numerical ranges described in this specification and claims are equivalent to describing at least each specific integer value therein, and should be understood to describe the two endpoints of the range, each integer within the range, and each decimal within the range.

### Beneficial Effects of the Invention

The inventors found that the compound of formula I has strong adhesiveness and is prone to aggregation during the dissolution process, which is not conducive to dissolution. Therefore, through experimental research, an IRAK4 inhibitor pharmaceutical composition with good dissolution performance, low impurity content, and good stability is provided.

### BRIEF DESCRIPTION OF THE DRAWINGS

FIG. 1 is the XRPD patterns of crystalline forms II and III.
FIG. 2 is a DSC-TGA pattern of crystalline form III.
FIG. 3 is the PLM pattern of crystalline form III (scale 2.5 µm).
FIG. 4 is the XRPD pattern of crystalline form VII.
FIG. 5 is a DSC-TGA pattern of crystalline form VII.
FIG. 6 is the PLM pattern of crystalline form VII (scale 5 µm).

### DETAILED DESCRIPTION

The technical solution of the present invention will be further described in detail below in conjunction with specific examples. It should be understood that the following examples are only intended to illustrate and explain the present invention by way of example only, and should not be construed as limiting the scope of protection of the present invention. All technologies achieved based on the above contents of the present invention are covered in the scope that the present invention is intended to protect.

Unless otherwise specified, all the raw materials and reagents used in the following examples are commercially available or can be prepared by known methods.

The active ingredient used in the examples is the following compound (chemical name: 2-((2-((1r,4r)-4-hydroxy-4-methylcyclohexyl)-6-methoxy-2H-indazol-5-yl)carbamoyl)-6-methylpyridine 1-oxide): 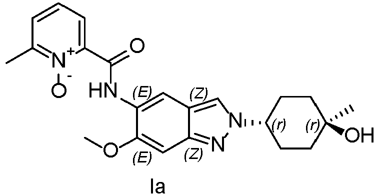 the compound can be prepared with reference to WO2021057785A1, and the crystalline forms of the compound (including crystalline form III and crystalline form VII) can be obtained with reference to CN202210267507.8.

### Example 1

Compositions were prepared according to the formulations shown in Table 1, and their dissolution rates were measured.

Preparation method: the active ingredient, sodium dodecyl sulfate, colloidal silicon dioxide, sodium carboxymethyl starch, magnesium stearate, and film coating premix (gastric soluble) Opadry were weighed; wherein the microcrystalline cellulose was divided into two portions and weighed; for formulation 1, lactose was divided into two portions and weighed, and for formulation 2, anhydrous calcium hydrogen phosphate was divided into two portions and weighed, wherein one portion was weighed at 1/3 of the formulation amount, and the other was weighed at 2/3 of the formulation amount. 2/3 of anhydrous calcium hydrogen phosphate, 1/2 of microcrystalline cellulose, sodium carboxymethyl starch, and magnesium stearate were passed through a 40-mesh sieve respectively for later use. The active ingredient, colloidal silicon dioxide, and sodium dodecyl sulfate were mixed, and then the mixture was passed through a 60-mesh sieve to obtain a premix material 1. Then, 1/2 of microcrystalline cellulose, 1/3 of anhydrous calcium hydrogen phosphate, and the premix material 1 were added into a hopper, mixed, and passed through a 60-mesh sieve to obtain a premix material 2. 1/2 of microcrystalline cellulose, the premix material 2, sodium carboxymethyl starch, and 2/3 of anhydrous calcium hydrogen phosphate were placed in sequence in a hopper mixer, and mixed. Magnesium stearate was added into the hopper mixer, and mixed for 3 min. The total mixed powder was placed in a tablet press for tableting.

**Table 1**

| | Formulation 1 | Formulation 2 |
|---|---|---|
| Compound of formula I | 10% | 10% |
| lactose | 58.67% | - |
| anhydrous calcium hydrogen phosphate | - | 63.75% |
| microcrystalline cellulose | 28.33% | 21.25% |
| colloidal silicon dioxide | 1% | 1% |
| cross-linked sodium carboxymethyl cellulose | 3% | 3% |
| magnesium stearate | 1% | 1% |

**Table 2**

| Formulation | Dissolution results (%) (n=3) | | | |
|---|---|---|---|---|
| | 15min | 30min | 45min | 60min |
| Formulation 1 | 28 | 36 | 42 | 50 |
| Formulation 2 | 66 | 82 | 88 | 92 |

It can be seen from Table 1 and Table 2 that since anhydrous calcium hydrogen phosphate facilitates the dispersion of the compound of formula I during the dissolution process, and anhydrous calcium hydrogen phosphate and microcrystalline cellulose serve as diluents, the dissolution effect of the formulation is better.

### Example 2

Formulation 3 and Formulation 4 were prepared according to the preparation method of Formulation 2. The formula ratios of Formulation 3 and Formulation 4 are shown in Table 3.

**Table 3**

| | Formulation 3 | Formulation 4 |
|---|---|---|
| compound of formula I | 10% | 10% |
| anhydrous calcium hydrogen phosphate | 63.75% | 62.25% |
| microcrystalline cellulose | 21.25% | 20.75% |
| colloidal silicon dioxide | 1% | 3% |
| cross-linked sodium carboxymethyl cellulose | 3% | 3% |
| magnesium stearate | 1% | 1% |

**Table 4**

| Formulation | Dissolution results (%) (n=3) | | | |
|---|---|---|---|---|
| | 15min | 30min | 45min | 60min |
| Formulation 3 | 66 | 82 | 88 | 92 |
| Formulation 4 | 45 | 65 | 75 | 82 |

It can be seen from Tables 3 and 4 that the dissolution effect of Formulation 3 is better.

### Example 3

Formulation 5, Formulation 6 and Formulation 7 were prepared according to the preparation method of Formulation 2. The formula ratios of Formulation 5, Formulation 6 and Formulation 7 are shown in Table 5.

**Table 5**

| | Formulation 5 | Formulation 6 | Formulation 7 |
|---|---|---|---|
| compound of formula I | 10% | 10% | 10% |
| anhydrous calcium hydrogen phosphate | 63% | 62.2% | 61.5% |
| microcrystalline cellulose | 21% | 20.8% | 20.5% |
| sodium dodecyl sulfate | 1% | 2% | 3% |
| colloidal silicon dioxide | 1% | 1% | 1% |
| cross-linked sodium carboxymethyl cellulose | 3% | 3% | 3% |
| magnesium stearate | 1% | 1% | 1% |

**Table 6**

| Formulation | Dissolution results (%) (n=3) | | | |
|---|---|---|---|---|
| | 15min | 30min | 45min | 60min |
| Formulation 5 | 60 | 79 | 90 | 96 |
| Formulation 6 | 65 | 84 | 90 | 95 |
| Formulation 7 | 72 | 89 | 95 | 98 |

As can be seen from Table 6, when sodium dodecyl sulfate is added to the formulation, the dissolution effect is always good.

### Example 4

Formulation 8 and Formulation 9 were prepared according to the preparation method of Formulation 2. The formula ratios of Formulation 8 and Formulation 9 are shown in Table 7.

**Table 7**

| | Formulation 8 | Formulation 9 |
|---|---|---|
| compound of formula I | 10% | 10% |
| anhydrous calcium hydrogen phosphate | 61.5% | 61.5% |
| microcrystalline cellulose | 20.5% | 20.5% |
| sodium dodecyl sulfate | 3% | 3% |
| colloidal silicon dioxide | 1% | 1% |
| cross-linked sodium carboxymethyl cellulose | 3% | - |
| sodium carboxymethyl starch | - | 3% |
| magnesium stearate | 1% | 1% |

**Table 8**

| Formulation | Dissolution results (%) (n=3) | | | |
|---|---|---|---|---|
| | 15min | 30min | 45min | 60min |
| Formulation 8 | 72 | 89 | 95 | 98 |
| Formulation 9 | 87 | 96 | 98 | 99 |

As shown in Table 8, adding sodium carboxymethyl starch as a disintegrant in the formulation achieves a better dissolution effect.

### Example 5

Formulation 10 and Formulation 11 were prepared according to the preparation method of Formulation 2.

**Table 9**

| | Formulation 10 | Formulation 11 |
|---|---|---|
| Plain tablets | | |
| compound of formula I | 10% | 10% |
| anhydrous calcium hydrogen phosphate | 61.5% | 61.5% |
| microcrystalline cellulose | 20.5% | 20.5% |
| sodium dodecyl sulfate | 3% | 3% |
| colloidal silicon dioxide | 1% | 1% |
| sodium carboxymethyl starch | 3% | 3% |
| magnesium stearate | 1% | 1% |
| Coating | | |
| Opadry II | 2% | - |
| Opadry I | - | 2% |

**Table 10**

| Formulation | Dissolution results (%) (n=3) | | | |
|---|---|---|---|---|
| | 15min | 30min | 45min | 60min |
| Opadry II | 72 | 86 | 91 | 94 |
| Opadry I | 83 | 95 | 98 | 99 |

As can be seen from Table 10, the dissolution effect is good when the coating is either Opadry I or II.

### Example 6

The stability factor of Formulation 11 is studied under various conditions, as shown in Table 11.

**Table 11**

| Placement conditions | Dissolution results (%) (n=3) | | | |
|---|---|---|---|---|
| | 15min | 30min | 45min | 60min |
| Day 0 | 87% | 94% | 94% | 95% |
| 40°C/75%RH (closed) - 10 days | 76% | 93% | 98% | 100% |
| 40°C/75%RH (closed + desiccant) - 10 days | 91% | 100% | 102% | 103% |
| 40°C/75%RH (closed + desiccant) - 30 days | 93% | 101% | 101% | 103% |
| Illumination | 87% | 96% | 98% | 99% |

As can be seen from Table 11, the dissolution of the composition does not change significantly under various conditions, and therefore, the composition is very stable under all conditions.

### Example 7

The total impurity content of Formulation 11 under various conditions is studied as shown in Table 12.

**Table 12**

| Placement conditions | Total impurity content (%) |
|---|---|
| Day 0 | 0.13% |
| 40°C/75%RH (open) - 10 days | 0.19% |
| 40°C/75%RH (open) - 30 days | 0.20% |
| 40°C/75%RH (closed) - 10 days | 0.21% |
| 40°C/75%RH (closed + desiccant) - 10 days | 0.22% |
| 40°C/75%RH (closed + desiccant) - 30 days | 0.25% |
| Illumination - 10 days | 0.94% |
| Illumination (closed + desiccant) - 10 days | 0.39% |

It can be seen from Table 12 that under the accelerated conditions of 40°C/75%RH, the impurities do not increase under the open, closed, and closed with desiccant conditions. Therefore, the composition has good stability.

### Example 8

The dissolution results of plain tablets of different hardness of Formulation 11 are shown in Table 13.

**Table 13**

| Hardness | Dissolution results (%) (n=3) | | | |
|---|---|---|---|---|
| | 15min | 30min | 45min | 60min |
| 40N | 88 | 94 | 96 | 97 |
| 70N | 89 | 95 | 96 | 97 |
| 100N | 90 | 96 | 98 | 98 |

As can be seen from Table 13, the hardness of the composition is in the range of 40-70N, and the dissolution is good.

The specific examples of the present invention have been described above. It should be understood that the present invention is not limited to the above examples, and the above examples and descriptions are only for illustrating the principles of the present invention. Those skilled in the art may make various non-substantial changes and improvements to the present invention without departing from the concept of the present invention, all of which fall within the scope of protection claimed by the present invention.

## Claims

1. A pharmaceutical composition, comprising:
(i) a compound of formula I, a stereoisomer, a solvate, or a pharmaceutically acceptable salt thereof, wherein the structure of formula I is shown as follows: 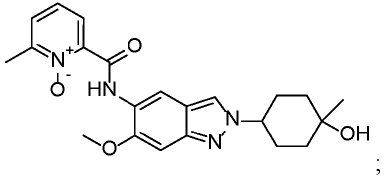
(ii) a diluent;
(iii) a disintegrant; and
(iv) a lubricant.

2. The pharmaceutical composition according to claim 1, **characterized in that** the composition may optionally further comprise at least one of (v) a glidant and (vi) a surfactant.

3. The pharmaceutical composition according to claim 1 or 2, **characterized in that** the diluent is selected from at least one of microcrystalline cellulose, anhydrous calcium hydrogen phosphate, and lactose; preferably, the diluent is selected from at least one of anhydrous calcium hydrogen phosphate and microcrystalline cellulose; more preferably, the diluent is a combination of anhydrous calcium hydrogen phosphate and microcrystalline cellulose, and even more preferably, the mass ratio of anhydrous calcium hydrogen phosphate to microcrystalline cellulose is 1:10 to 10:1; preferably, based on the total weight of the composition, the content of the diluent ranges from 18% to 90%.

4. The pharmaceutical composition according to any one of claims 1 to 3, **characterized in that** the glidant is selected from colloidal silicon dioxide; preferably, based on the total weight of the composition, the content of the glidant ranges from 0.1% to 5%.

5. The pharmaceutical composition according to any one of claims 1 to 4, **characterized in that** the disintegrant is selected from at least one of cross-linked sodium carboxymethyl cellulose, sodium carboxymethyl starch, hydroxypropyl cellulose, cross-linked polyvinyl pyrrolidone, and cross-linked povidone, preferably at least one of cross-linked sodium carboxymethyl cellulose and sodium carboxymethyl starch, more preferably sodium carboxymethyl starch; preferably, based on the total weight of the composition, the content of the disintegrant ranges from 1% to 10%.

6. The pharmaceutical composition according to any one of claims 1 to 5, **characterized in that** the surfactant is selected from at least one of sodium dodecyl sulfate, Tween 80, poloxamer, and potassium oleate, preferably sodium dodecyl sulfate; preferably, based on the total weight of the composition, the content of the surfactant ranges from 1% to 5%.

7. The pharmaceutical composition according to any one of claims 1 to 6, **characterized in that** the lubricant is selected from at least one of magnesium stearate, talc, sodium stearyl fumarate, zinc stearate, sodium lauryl sulfate, and hydrogenated vegetable oil, preferably magnesium stearate; preferably, based on the total weight of the composition, the content of the lubricant ranges from 0.1% to 5%.

8. The pharmaceutical composition according to any one of claims 1 to 7, **characterized in that** the compound of formula I is selected from the following structure: 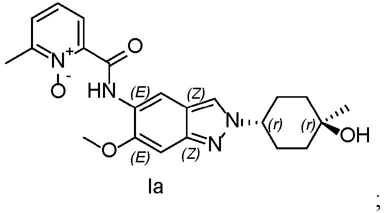
preferably, the compound of formula I is in the form of crystalline form III, and the crystalline form III has the X-ray powder diffraction expressed as 2θ angles comprising characteristic peaks at 12.15±0.20°, 15.98±0.20°, 16.62±0.20°, 17.14±0.20°, 24.32±0.20°, and 26.08±0.20° using Cu-Kα radiation;
preferably, the crystalline form III has the X-ray powder diffraction expressed as 2θ angles comprising characteristic peaks at 12.15±0.20°, 15.04±0.20°, 15.98±0.20°, 16.62±0.20°, 17.14±0.20°, 21.09±0.20°, 24.32±0.20°, and 26.08±0.20° using Cu-Kα radiation;
preferably, the crystalline form III has the X-ray powder diffraction expressed as 2θ angles comprising characteristic peaks at 12.15±0.20°, 15.04±0.20°, 15.98±0.20°, 16.62±0.20°, 17.14±0.20°, 18.74±0.20°, 21.09±0.20°, 23.51±0.20°, 24.32±0.20°, and 26.08±0.20°using Cu-Kα radiation;
preferably, the crystalline form III has the X-ray powder diffraction expressed as 2θ angles and shown in Table 1 using Cu-Kα radiation, with an error range of ±0.20°:
| Peak No. | 2θ [ °] | Relative Intensity % | Peak No. | 2θ [ °] | Relative Intensity % |
|---|---|---|---|---|---|
| 1 | 6.021 | 5.2 | 22 | 26.083 | 37.3 |
| 2 | 10.879 | 8.2 | 23 | 26.688 | 1.3 |
| 3 | 12.152 | 77.4 | 24 | 27.106 | 1 |
| 4 | 12.978 | 3.6 | 25 | 27.83 | 5.8 |
| 5 | 15.04 | 20.5 | 26 | 28.262 | 0.2 |
| 6 | 15.986 | 47.8 | 27 | 29.314 | 9.1 |
| 7 | 16.617 | 34.2 | 28 | 29.824 | 3.2 |
| 8 | 17.141 | 27 | 29 | 30.429 | 3.6 |
| 9 | 18.323 | 1.5 | 30 | 30.818 | 1.5 |
| 10 | 18.742 | 13.7 | 31 | 31.926 | 4.9 |
| 11 | 20.068 | 8.7 | 32 | 32.373 | 3.1 |
| 12 | 20.449 | 3.5 | 33 | 32.583 | 2.3 |
| 13 | 20.765 | 3.2 | 34 | 33.186 | 1.4 |
| 14 | 21.092 | 21.8 | 35 | 33.777 | 2.5 |
| 15 | 21.645 | 2.2 | 36 | 34.392 | 0.6 |
| 16 | 22.262 | 5.1 | 37 | 35.418 | 2.4 |
| 17 | 22.97 | 0.4 | 38 | 36.023 | 1.7 |
| 18 | 23.51 | 19.6 | 39 | 36.6 | 2 |
| 19 | 24.048 | 100 | 40 | 38.138 | 4 |
| 20 | 24.323 | 50.8 | 41 | 38.806 | 4.3 |
| 21 | 24.598 | 8.3 | 42 | 39.306 | 2 |
preferably, the crystalline form III has a powder X-ray diffraction pattern substantially as shown in FIG. 1;
preferably, differential scanning calorimetry (DSC) analysis of the crystalline form III shows that a first endothermic peak appears when being heated near the peak temperature of 188.81°C;
preferably, thermogravimetric analysis (TGA) of the crystalline form III shows almost no weight loss before 180°C;
preferably, the crystalline form III has a DSC-TGA pattern substantially as shown in FIG. 2;
preferably, the compound of formula I is in the form of crystalline form VII, and the crystalline form VII has the X-ray powder diffraction expressed as 2θ angles comprising characteristic peaks at 12.94±0.20°, 14.41±0.20°, 15.64±0.20°, 17.25±0.20°, 21.75±0.20°, and 24.23±0.20°using Cu-Kα radiation;
preferably, the crystalline form VII has the X-ray powder diffraction expressed as 2θ angles comprising characteristic peaks at 12.94±0.20°, 13.18±0.20°, 14.41±0.20°, 15.64±0.20°, 17.25±0.20°, 21.75±0.20°, 22.54±0.20°, and 24.23±0.20° using Cu-Kα radiation;
preferably, the crystalline form VII has the X-ray powder diffraction expressed as 2θ angles comprising characteristic peaks at 12.94±0.20°, 13.18±0.20°, 14.41±0.20°, 15.64±0.20°, 17.25±0.20°, 21.11±0.20°, 21.75±0.20°, 22.54±0.20°, 24.23±0.20°, 26.62±0.20°, and 31.64±0.20°using Cu-Kα radiation;
preferably, the crystalline form VII has the X-ray powder diffraction expressed as 2θ angles and shown in Table 2 using Cu-Kα radiation, with an error range of ±0.20°:
**Table 2 XRPD analysis data of crystalline form VII**
| Peak No. | 2θ [ ° ] | Relative Intensity % | Peak No. | 2θ [ ° ] | Relative Intensity % |
|---|---|---|---|---|---|
| 1 | 6.413 | 8 | 25 | 25.82 | 1.7 |
| 2 | 10.957 | 1.1 | 26 | 26.135 | 6.1 |
| 3 | 11.625 | 3.1 | 27 | 26.385 | 3 |
| 4 | 12.939 | 25.9 | 28 | 26.621 | 10.5 |
| 5 | 13.176 | 12.7 | 29 | 27.328 | 0.2 |
| 6 | 13.451 | 5.9 | 30 | 27.618 | 9.2 |
| 7 | 14.409 | 16.3 | 31 | 28.436 | 2 |
| 8 | 14.567 | 9.9 | 32 | 28.724 | 4.6 |
| 9 | 15.644 | 41.4 | 33 | 29.431 | 1.4 |
| 10 | 17.246 | 18.2 | 34 | 29.825 | 5.9 |
| 11 | 17.522 | 6.2 | 35 | 30.35 | 4.9 |
| 12 | 17.655 | 4.4 | 36 | 30.624 | 0.8 |
| 13 | 18.783 | 1.4 | 37 | 31.637 | 11.1 |
| 14 | 19.255 | 100 | 38 | 32.123 | 2.6 |
| 15 | 19.732 | 3.4 | 39 | 32.856 | 1.5 |
| 16 | 19.94 | 5.1 | 40 | 33.763 | 3.9 |
| 17 | 21.106 | 10.3 | 41 | 34.092 | 1.4 |
| 18 | 21.75 | 41 | 42 | 34.973 | 4.4 |
| 19 | 22.001 | 3.4 | 43 | 35.367 | 2.8 |
| 20 | 22.539 | 12.6 | 44 | 35.841 | 2.9 |
| 21 | 24.231 | 18 | 45 | 37.7 | 1.2 |
| 22 | 24.704 | 0.7 | 46 | 38.242 | 1.6 |
| 23 | 25.058 | 3.6 | 47 | 39.185 | 2.1 |
| 24 | 25.308 | 7 | 48 | 39.307 | 2.1 |
preferably, the crystalline form VII has a powder X-ray diffraction pattern substantially as shown in FIG. 4;
according to an embodiment of the present invention, differential scanning calorimetry (DSC) analysis of the crystalline form VII shows that an endothermic peak appears when being heated near the peak temperature of 201.07°C;
according to an embodiment of the present invention, thermogravimetric analysis (TGA) of the crystalline form VII shows almost no weight loss before 200°C, for example, almost no weight loss before 180°C; and
preferably, the crystalline form VII has a DSC-TGA pattern substantially as shown in FIG. 5.

9. The pharmaceutical composition according to any one of claims 1 to 8, **characterized in that** the pharmaceutical composition comprises the following components by weight percentage:
the compound of formula I 5-15%
anhydrous calcium hydrogen phosphate 55-65%
microcrystalline cellulose 15-25%
sodium dodecyl sulfate 0.5-5%
colloidal silicon dioxide 0.5-2%
cross-linked sodium carboxymethyl cellulose or sodium carboxymethyl starch 1-5%
magnesium stearate 0.5-5%.

10. The pharmaceutical composition according to any one of claims 1 to 9, **characterized in that** the pharmaceutical composition comprises the following components by weight percentage:
the compound of formula I 8-12%
anhydrous calcium hydrogen phosphate 60-65%
microcrystalline cellulose 18-25%
sodium dodecyl sulfate 0.5-3%
colloidal silicon dioxide 0.5-1.5%
cross-linked sodium carboxymethyl cellulose 2.5-3.5%
magnesium stearate 0.5-1.5%.

11. The pharmaceutical composition according to any one of claims 1 to 9, **characterized in that** the pharmaceutical composition comprises the following components by weight percentage:
the compound of formula I 8-12%
anhydrous calcium hydrogen phosphate 60-63%
microcrystalline cellulose 18-25%
sodium dodecyl sulfate 2.5-3.5%
colloidal silicon dioxide 0.5-1.5%
sodium carboxymethyl starch 2.5-3.5%
magnesium stearate 0.5-1.5%.

12. The pharmaceutical composition according to claim 10, **characterized in that** the pharmaceutical composition comprises the following components by weight percentage:
the compound of formula I 10%
anhydrous calcium hydrogen phosphate 63%
microcrystalline cellulose 21%
sodium dodecyl sulfate 1%
colloidal silicon dioxide 1%
cross-linked sodium carboxymethyl cellulose 3%
magnesium stearate 1%.

13. The pharmaceutical composition according to claim 11, **characterized in that** the pharmaceutical composition comprises the following components by weight percentage:
the compound of formula I 10%
anhydrous calcium hydrogen phosphate 61.5%
microcrystalline cellulose 20.5%
sodium dodecyl sulfate 3%
colloidal silicon dioxide 1%
sodium carboxymethyl starch 3%
magnesium stearate 1%.

14. The pharmaceutical composition according to any one of claims 1 to 13, **characterized in that** the oral preparation is selected from tablets, capsules, mini-tablets, or granules, preferably tablets; preferably, the tablet comprises a core and a coating, and the coating material is selected from at least one of hydroxypropyl methylcellulose, polyvinyl alcohol, hydroxypropyl cellulose, polyacrylic acid resin, and Opadry, more preferably, Opadry (for example, Opadry I and Opadry II); preferably, the mass of the coating accounts for 1-5% of the total mass of the tablet, preferably 1-4%, more preferably 2%.

15. A preparation method of the pharmaceutical composition according to any one of claims 1 to 14, **characterized in that** the method comprises: mixing the compound of formula I with other components and tableting; preferably, the preparation method further comprises a coating step.

16. The preparation method of the pharmaceutical composition according to claim 15, **characterized in that** the preparation method comprises the following steps:
(1) passing a disintegrant, a lubricant, and a portion of a diluent through a 20-60-mesh sieve;
(2) passing the compound of formula I or optionally the compound together with a glidant and a surfactant through a 40-100-mesh sieve to obtain a premix material 1; then adding the remaining diluent and the premix material 1 into a hopper, mixing, and passing the mixture through a 40-100-mesh sieve to obtain a premix material 2;
(3) placing, in sequence, the disintegrant, the diluent, and the premix material 2 that have passed through the 20-60-mesh sieve in a hopper mixer and mixing;
(4) adding a lubricant that has passed through a 20-60-mesh sieve into the hopper mixer and mixing; and
(5) placing the total mixed powder in a tablet press for tableting.

17. Use of the pharmaceutical composition according to any one of claims 1 to 14 in the preparation of a medicament for preventing and/or treating an IRAK-mediated disease or condition, wherein preferably, the IRAK-mediated disease or condition is selected from diseases such as tumors, gout, systemic lupus erythematosus, multiple sclerosis, metabolic syndrome, atherosclerosis, myocardial infarction, pyaemia, inflammatory bowel disease, rheumatoid arthritis, asthma, and allergies.

18. Use of the pharmaceutical composition according to any one of claims 1 to 14 in the preparation of a medicament for preventing and/or treating an interleukin-1 receptor-associated kinase mediated disease or condition, wherein preferably, the interleukin-1 receptor-associated kinase mediated disease or condition is selected from diseases such as tumors, gout, systemic lupus erythematosus, multiple sclerosis, metabolic syndrome, atherosclerosis, myocardial infarction, sepsis, inflammatory bowel disease, asthma, rheumatoid arthritis, psoriasis, pyaemia, autoimmune diseases, and allergies.
